(19)

Europäisches
Patentamt
European
Patent Office
Office européen
des brevets

(11) **EP 4 428 865 A1**

(12) **EUROPEAN PATENT APPLICATION**
published in accordance with Art. 153(4) EPC

(43) Date of publication:
**11.09.2024 Bulletin 2024/37**

(21) Application number: **23880139.3**

(22) Date of filing: **16.10.2023**

(51) International Patent Classification (IPC):
*G16C 20/80* (2019.01)    *G16C 20/90* (2019.01)
*G16C 20/30* (2019.01)    *G16C 20/70* (2019.01)
*G06N 3/08* (2023.01)

(52) Cooperative Patent Classification (CPC):
**G06N 3/08; G16C 20/30; G16C 20/70; G16C 20/80;
G16C 20/90**

(86) International application number:
**PCT/KR2023/015926**

(87) International publication number:
**WO 2024/085562 (25.04.2024 Gazette 2024/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.10.2022  KR 20220134831
13.10.2023  KR 20230136974**

(71) Applicant: **LG Chem, Ltd.
Yeongdeungpo-gu
Seoul 07336 (KR)**

(72) Inventors:
• **MOON, Hee Yeon**
  **Daejeon 34122 (KR)**
• **BAE, Jae Soon**
  **Daejeon 34122 (KR)**
• **LEE, Kyu Hwang**
  **Daejeon 34122 (KR)**
• **SHIN, Hyeon Ah**
  **Daejeon 34122 (KR)**

(74) Representative: **Hoffmann Eitle
Patent- und Rechtsanwälte PartmbB
Arabellastraße 30
81925 München (DE)**

(54) **POLYMER GRAPH NEURAL NETWORK AND IMPLEMENTATION METHOD THEREFOR**

(57)    The present invention provides a computer-implemented method for predicting property information of a polymer from a chemical structure of the polymer. The present invention provides a method for graphically representing the chemical structure of the polymer, and provides a method and system for producing property information of a polymer from graph information of the polymer by performing machine learning on the chemical structure of the polymer based on information prescribing an interconnection relationship between respective atoms constituting a repeat unit structure of the polymer and an attach node to which the repeat unit structure is attached.

FIG. 5

**Description**

**TECHNICAL FIELD**

The present invention relates to a method and system for representing a structure of a polymer so that the structure of the polymer can be recognized by a computer device.

**BACKGROUND ART**

In order to input a structure of a compound into the computer device and perform predetermined processing, various methods of representing the compound structure have been proposed so that the compound structure polymer can be recognized by the computer device. Typically, there was SMILES, which represents the structure of the compound as a string, or a way of representing the string converted into SMILES representation as a descriptor.

However, it is difficult to represent a polymer that is not monomers in the SMILES representation method, and thus BigSMILES or Hierarchical Descriptor were proposed.

However, these technologies of prior art had problems such as the larger the molecule, the more complex its representation method became, only representing the polymer fragmentarily and lacking technology to process molecules based on the representation, not being able to properly represent the properties of the polymer in which repeat unit structures are infinitely repeated.

The technologies of prior art related to this are as follows.

Patent Document 1: Korean unexamined patent application publication No. 2021-0042777 A
Patent Document 2: Korean unexamined patent application publication No. 2021-0110539 A

**DISCLOSURE OF THE INVENTION**

**TECHNICAL PROBLEM**

In order to solve the problem described above, the present invention is intended to provide a data structure that represents a polymer material as graphic information, and further provide a method and system for predicting predetermined properties of a polymer using the data structure that represents graphic information of the polymer.

**TECHNICAL SOLUTION**

In order to solve the problems of the prior art described above, the present invention provides, in a method for converting a chemical structure data of a polymer into data so as to be able to be processed by a computer, a data processing method of polymer chemical structure including a polymer node attach variable setting step of setting an interconnection relationship between nodes representing respective atoms constituting a repeat unit structure of a selected polymer and attach nodes attached to an attach point, which is a point to which the repeat unit structure is repeatedly attached, among the nodes as a polymer node attach variable, and a polymer graph node attach data allocation step of allocating an attribute value of the interconnection relationship between the nodes and the attach nodes to the set polymer node attach variable.

The data processing method of polymer chemical structure of the present invention may further include a polymer node attribute variable setting step of setting attribute information of the node corresponding to constituent atoms of the repeat unit structure forming the selected polymer and information of the attach node attached to the attach point, which is the point to which the repeat unit structure is repeatedly attached, among the nodes as a polymer node attribute variable, a polymer graph node attribute information allocation step of allocating an attribute value of the nodes and attach nodes to the set polymer node attribute variable, a polymer node attribute variable setting step of setting the attribute information of the node corresponding to respective atoms constituting the repeat unit structure forming the selected polymer and the information of the attach node attached to the attach point, which is the point to which the repeat unit structure is repeatedly attached, among the nodes as the polymer node attribute variable, a polymer graph node attribute information allocation step of allocating attribute values of the nodes and attach nodes to the set polymer node attribute variable, a polymer edge attribute variable setting step of setting information of an edge interconnecting nodes corresponding to respective atoms constituting the repeat unit structure forming the selected polymer and an attach edge connecting at least one of the nodes and the attach node as a polymer edge attribute variable, and a polymer graph edge attribute information allocation step of allocating attribute values of the edges and attached edges to the set polymer edge attribute variable.

The present invention also provides, as a method for generating a computer-implemented model that graphically describes a chemical structure of a selected polymer and analyzes predetermined properties of the polymer, a generation method of polymer property analysis model including a basic data acquisition step of acquiring basic data consisting of chemical structure information of a plurality of learning polymers and known values of predetermined property information of the corresponding polymers, a data pre-processing process of converting the chemical structure information of the corresponding learning polymer among the basic data into polymer graph data, a polymer property information prediction artificial neural network construction step of constructing a polymer property information prediction artificial neural network so as to receive the polymer graph data and output a predetermined property information prediction value of the polymer, a training data input step of inputting the polymer graph data of the learning polymer and the predetermined property information of the learning polymer into the polymer property information prediction artificial neural network, and a learning step of the polymer property information prediction artificial neural network for updating parameters of the polymer property information prediction artificial neural network, based on a comparison result between the predetermined property information prediction value of the learning polymer output by the polymer property information prediction artificial neural network and the known value of the predetermined property information of the learning polymer. In this case, the polymer graph data includes at least one of polymer graph node attach data which is information representing an interconnection relationship between nodes corresponding to respective atoms constituting a repeat unit structure forming the polymer and attach nodes attached to an attach point, which is a point to which the repeat unit structure is repeatedly attached, among the nodes, polymer node attribute information, which is information representing attribute information of the node corresponding to respective atoms constituting the repeat unit structure forming the polymer, and representing an attribute of the attach nodes attached to an attach point, which is a point to which the repeat unit structure is repeatedly attached, among the nodes, and polymer edge attribute information, which is information representing the attributes of an edge interconnecting the nodes corresponding to respective atoms constituting the repeat unit structure forming the polymer, and an attach edge connecting at least one of the nodes and the attach node.

The present invention provides a computer-implemented method for graphically describing a chemical structure of a selected polymer and analyzing predetermined properties of the polymer, the computer-implemented method including a polymer property information production artificial neural network construction step of generating an artificial neural network that is subjected to machine learning so as to predict predetermined properties of the selected polymer based at least in part on chemical structure data related to the polymer, a polymer graph data acquisition step of acquiring polymer graph data that describes the chemical structure of the selected polymer as graphic data, a polymer graph input step of providing the acquired polymer graph data as an input to the graph neural network subjected to machine learning, and a step of receiving prediction data describing the predetermined properties of the selected polymer as an output of the graph neural network subjected to machine learning, in which the polymer graph data that describes the chemical structure of the selected polymer as graphic data includes at least one of polymer graph node attach data which is information representing an interconnection relationship between nodes corresponding to respective atoms constituting a repeat unit structure forming the polymer and attach nodes attached to an attach point, which is a point to which the repeat unit structure is repeatedly attached, among the nodes, polymer node attribute information, which is information representing attribute information of the node corresponding to respective atoms constituting the repeat unit structure forming the polymer, and an attribute of the attach node attached to the attach point, which is a point to which the repeat unit structure is repeatedly attached, among the nodes, and polymer edge attribute information, which is information representing attributes of an edge interconnecting nodes corresponding to respective atoms constituting the repeat unit structure forming the polymer, and an attach edge connecting at least one of the nodes and the attach node.

In this case, the step of providing the acquired polymer graph data as the input to the graph neural network subjected to machine learning may further include a polymer adjacency matrix construction step of constructing an adjacency matrix representing an interconnection relationship between two or more nodes representing respective atoms constituting the single molecule, and a polymer adjacency matrix input step of inputting the constructed adjacency matrix into the graph neural network, and the polymer adjacency matrix may further include connection relationship information between the attach node and the nodes.

The present invention also provides a system as a computer device implementing the method described above.

## ADVANTAGEOUS EFFECTS

According to the present invention, by providing a data structure that allows the polymer to be represented in graphical information that can be used by a computer, it is possible to implement a method and system for predicting property information of polymer materials by performing machine learning on a relationship between a molecular structure and the property information of the polymer materials through the graph neural network (GNN) and the message passing neural network (MPNN). In addition, it has been possible to provide a method and system for predicting polymer property information with improved accuracy compared to the prior art.

**BRIEF DESCRIPTION OF THE DRAWINGS**

The following drawings attached to this specification illustrate preferred embodiments of the present invention, and serve to further understand the technical idea of the present invention together with the detailed description of the invention described above. Therefore, the present invention should not be construed as limited to the matters described in such drawings.

FIG. 1 is a diagram illustrating a graphical representation of monomers and polymers according to the prior art.
FIG. 2 is a diagram illustrating a graphical representation of a polymer according to the present invention.
FIG. 3 is an exemplary diagram for describing the graphical representation of the polymer according to the present invention.
FIG. 4 is a diagram illustrating a progress of message passing-based learning according to the graphical representation of the polymer according to the present invention.
FIG. 5 is a diagram illustrating a procedure for implementing an artificial neural network that produces property information of a polymer using the graphical representation of the polymer according to the present invention.
FIG. 6 is a diagram illustrating a polymer property information prediction system according to the present invention.
FIG. 7 is a diagram illustrating an example of polymer graph informatization according to the prior art and the present invention.
FIG. 8 is a diagram illustrating another example of polymer graph informatization according to the prior art and the present invention.

**MODE FOR CARRYING OUT THE INVENTION**

1. Graph representation method of polymer according to the present invention

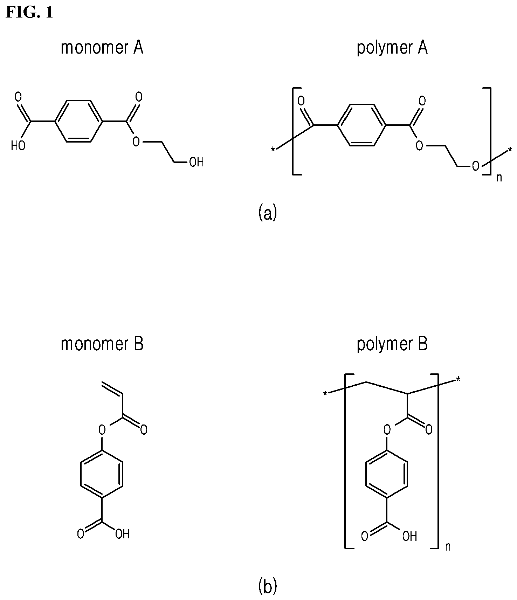

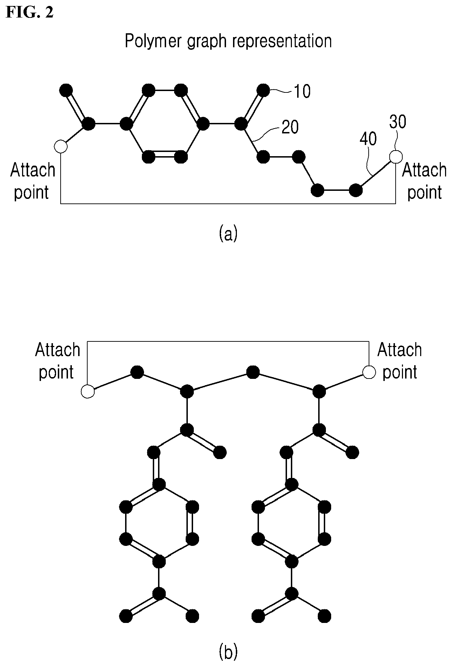

First, a method for graphically representing a polymer according to the present invention will be described with reference to FIGS. 1 and 2.
The (a) of and (b) FIG. 1 are diagrams illustrating the structures of different polymers and monomers that make up each polymer. However, when trying to process molecular structure data using an artificial neural network, especially when representing a molecular structure in a graph for application to GNN, etc., there was no method for effectively representing the chemical structure of the polymer in a graph.
Although the monomers that form the polymer illustrated in (a) and (b) of FIG. 1 are different from each other, since they are identical except for some of the monomer ends, the monomer graphs are represented very similarly. Therefore, it was inaccurate to represent the polymer graph simply as a monomer graph and to represent the polymer graph as a repetition of the monomer graph.
Therefore, the present invention proposes a new polymer graph representation method as illustrated in FIG. 2. Hereinafter, the polymer graph data representation method according to the present invention will be described with reference to FIG. 2.
First, in the polymer graph representation method of the present invention, the monomers that make up the polymer and repeat unit structures that repeat to make up the polymer are derived. FIG. 1 illustrates an example of a conventional polymer representation and an indication of the monomers constituting the polymer, and FIG. 2 is a diagram illustrating the repeat unit structures that make up the polymer of FIG. 1. In the repeat unit structure of FIG. 2, for the polymer representation method of the present invention, each atom is represented as a node 10, and a bond between the atoms is represented as an edge 20. Next, when the repeat unit structure, which is one of the features of the present invention, is repeated to form a polymer, a point to which the repeat unit structure is repeatedly attached is represented as an attach point 30, a node to which the attach point is attached is represented as an 'attach node'. In addition, the attach point 30 is a virtual representation of the attach node of a neighboring repeat unit structure that is repeatedly attached, and the connection connecting the attach point and the attach node is represented as an attach edge 40.
According to this polymer graph representation method of the present invention, when training an artificial neural network model based on the chemical structure of the polymer, especially, when learning is conducted by applying the artificial neural network model of the message passing-based message passing neural network (MPNN) and graph neural network (GNN), learning in which information from the opposite node to which the monomer is connected is transmitted just by adding minimal information in addition to the monomer structure information becomes possible, and learning that reflects the unit repetition feature of the polymer becomes possible.

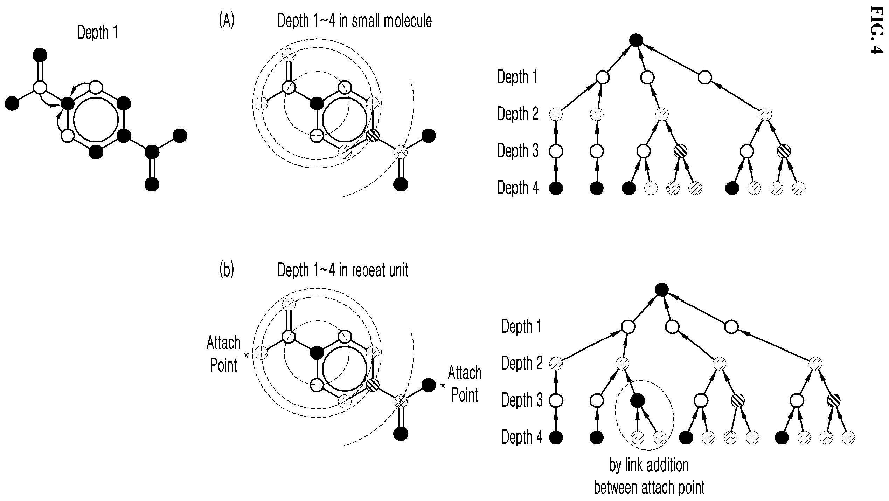

FIG. 4 is an example of representing structural information of monomers and polymers in the form of a binary tree, and illustrates that information of the opposite node is included by adding an attach point tree, as illustrated in (b) of FIG. 4, in addition to the monomer binary tree of (A) of FIG. 4.

2. Computer-usable datafication of graphical representations of polymers according to the present invention

The present invention converts the chemical structure of the polymer into a data structure that can be used in computer processing, that is, 'graph data', according to a graph representation method that graphically represents the molecular structure or chemical structure of the polymer described above. In the present invention, this is referred to as 'polymer graph data'.

The polymer graph data that describes the chemical structure of a predetermined or selected polymer in graphic information according to the present invention is configured to include at least one piece of information among node data, which is nodes 10 representing two or more nodes representing respective atoms constituting a repeat unit structure of monomers constituting the polymer and data related to the node 10, edge data, which is data related to one or more edges 20 representing the bond between respective nodes, attaching node data which is data related to at least one attach node, which is a point to which the repeat unit structures forming the polymer are repeatedly attached, among the nodes, and attach edge data, which is data related to an attach edge 40, which is a connection connecting the attach point 30 and the attach node.

The difference between monomer graph data and the polymer graph data of the present invention is that the polymer graph data of the present invention represents the node 10 attached to the attach point 30 and its edge data as the attach node and the attach edge in the graph data representation of the repeat unit structure including the monomer graph data.

2.1. Adjacency matrix and Feature matrix

When converting a graph structure of the monomer into data that can be used on a computer, there may be various data representation methods, but usually the edge data and the node data can be represented as the adjacency matrix and the feature matrix, respectively. The edge data is information related to the bond between atoms, and the node data is information related to each atom.

According to a polymer representation method according to the present invention, in addition to the conventional monomer graph representation method, the polymer graph can be converted into computer-recognizable data by additionally adding information of the node attached to the attach point 30 and attach edge 40.

(1) Adjacency matrix representation of monomer graph

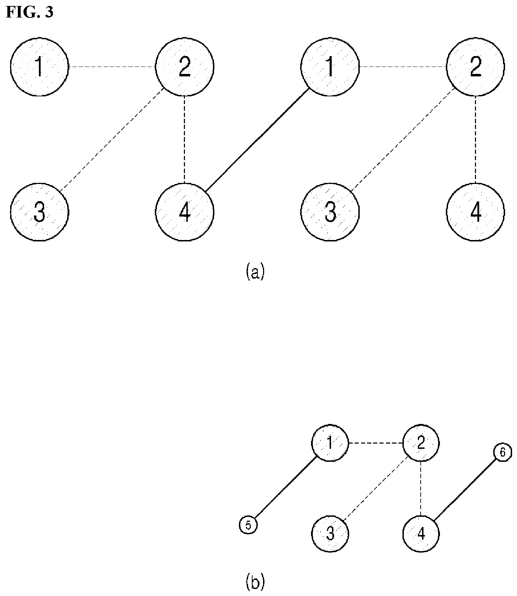

For the sake of simplicity of explanation, if the adjacency matrix representation of the monomer graph is explained using a simple structure as illustrated in FIG. 3, an adjacency matrix A of the monomer repeat unit structure composed of nodes 1, 2, 3, and 4 can be represented as follows.

$$\text{monomer adjacency matrix A} = \begin{bmatrix} 0 & 1 & 0 & 0 \\ 1 & 0 & 1 & 1 \\ 0 & 1 & 0 & 0 \\ 0 & 1 & 0 & 0 \end{bmatrix}$$

The adjacency matrix A has as many rows and columns as the number of nodes, and each component $A_{i,j}$ is information representing whether the i-th node (i = 1, ..., 4) and the j-th node (j = 1, ..., 4) are connected to each other. The value of each matrix element represents information on connection with other nodes. For example, since node 1 is connected only to node 2 and not to nodes 1, 3, and 4, it is represented as $A_{i,j}(j = 1, ..., 4) = [0\ 1\ 0\ 0]$ in the adjacency matrix.

(2) Adjacency matrix representation of the polymer graph according to the present invention

According to the polymer graph data representation method of the present invention, the polymer adjacency matrix PA, which is an adjacency matrix representation of the polymer graph according to the example of FIG. 3, can be represented as follows.

$$\text{polymer adjacency matrix PA} = \begin{bmatrix} 0 & 1 & 0 & 1 \\ 1 & 0 & 1 & 1 \\ 0 & 1 & 0 & 0 \\ 1 & 1 & 0 & 0 \end{bmatrix}$$

As illustrated in FIG. 3, the polymer adjacency matrix PA has two attach points (5, 6) added and these attach points refer to nodes 4 and 1 of adjacent monomers, respectively, and thus, nodes 1 and 4 are represented as connected to nodes 4 and 1, respectively. That is, since there is no node connected to nodes 1 and 4 in the monomer, matrix values indicating the connection relationship between nodes 1 and 4 were $A_{1,4} = 0$, $A_{4,1} = 0$, but, in the polymer representation method of the present invention, since nodes 1 and 4 are connected to nodes 4 and 1 of adjacent monomers, respectively, and thus, in the polymer adjacency matrix PA, the matrix values indicating the connection relationship between nodes 1 and 4 are represented as $PA_{1,4} = 1$, $PA_{4,1} = 1$, and thus repetitive attach data of the polymer are included. That is, nodes 1 and 4, which were not connected in the monomer, are connected to each other in the polymer, and thus they are represented as being connected to each other in the polymer adjacency matrix in which the attach edge data is represented.

However, in this case, since nodes 1 and 4 within the monomer are not connected to each other, in order to represent that it is not a connection within a monomer, but a connection with the node of an adjacent repeat unit structure, they can be distinguished by representing the corresponding node connection relationship as a negative number to represent the polymer adjacency matrix as follows, assigning an 'attach node' feature connected to the attach point to the feature of the node in the node feature matrix described later, or assigning an adjacent repeat unit structure attach attribute to the edge feature matrix.

$$\text{polymer adjacency matrix PA'} = \begin{bmatrix} 0 & 1 & 0 & -1 \\ 1 & 0 & 1 & 1 \\ 0 & 1 & 0 & 0 \\ -1 & 1 & 0 & 0 \end{bmatrix}$$

In other words, graph data, which graphs the chemical structure of the polymer according to the present invention and describes it as graphic data, also includes, in addition to the graph data of the monomer repeat unit structure, attaching node data representing the attach node, which is a node to which monomers are repeatedly attached, and information of the attach edge 40 connected to the attach node.

According to the representation method of the polymer adjacency matrix PA above, it is represented that the monomer repeat unit structure composed of nodes 1, 2, 3, and 4 is repeated by being connected to nodes 4 and 1 of the neighboring repeat unit structure at its nodes 1 and 4, respectively. However, in this case, since nodes 1 and 4 in FIG. 3 are not connected to each other within the monomer, but are connected to the nodes of adjacent repeat unit structures, these nodes can be distinguished by designating them as 'attach nodes' and assigning an attach node attribute to the node feature matrix, or assigning an adjacent repeat unit structure attach attribute to the edge feature matrix.

This can be used as an embedding procedure in order to convert the chemical structure of a polymer into data that can be processed by a computer, and is achieved through a polymer edge variable setting step of setting the interconnection relationship between nodes representing respective atoms constituting the monomer repeat unit structure of the selected polymer and attach nodes, which are points to which the monomer is repeatedly attached, as a polymer attach edge variable, and a polymer graph edge data allocation step of allocating attribute values of the interconnection relationships of the nodes and the attach nodes to the set polymer attach edge variable. As seen in the polymer adjacency matrix PA above, the attribute value of the interconnection relationship between the nodes and the attach nodes can be determined as '1' or '0', indicating connection/non-connection, and this is represented as a matrix, which is the polymer adjacency matrix. The polymer adjacency matrix of the present invention has the same component values as a typical adjacency matrix, but as described above, it varies by adding attach nodes to a typical single molecule graph node.

(3) Feature matrix of monomer graph

The graph representation of a compound may have, in addition to the adjacency matrix illustrating the connection relationship between nodes, a feature matrix representing predetermined attribute information of each node and predetermined attribute information of each edge as graph data. In the exemplary monomer graph of FIG. 3, the monomer node feature matrix containing attribute value information (e.g., three attribute information) of each atom can be represented as follows.

$$\text{monomer node feature matrix NF} = \begin{bmatrix} 0 & 1 & 0 \\ 1 & 0 & 1 \\ 0 & 1 & 0 \\ 0 & 1 & 0 \end{bmatrix}$$

The monomer node feature matrix NF has as many rows as the number of each node and as many columns as the number of types of feature values to be represented for each node, and thus can represent a predetermined attribute to be represented for each atom. Each component $NF_{i,j}$ has a j-th feature value of an i-th node. For explanation purposes, the monomer node feature matrix NF is described as an example in which each node has three arbitrary attribute values.

The information of the monomer graph can be represented as an edge feature matrix EF, where each row represents each edge of a monomer, and the column data of each row can be represented so as to represent a predetermined attribute value of each edge. Edge attribute values may include types of chemical bonds (single bond, double bond, etc.), ring status, conjugation status, etc. For example, in the case of the monomer in FIG. 3, there are three attach edges, and thus the edge feature matrix has three rows, and edge attribute values to contain information are allocated to each column. An example of an edge feature matrix with three arbitrary edge attribute values is as follows.

$$\text{monomer edge feature matrix EF} = \begin{bmatrix} 1 & 0 & 0 \\ 0 & 1 & 1 \\ 0 & 0 & 0 \end{bmatrix}$$

(4) Feature matrix of polymer graph according to the present invention

Polymer graph data according to the present invention may have separate attribute values added to the attribute values of the monomer node feature matrix described above. For example, the polymer feature matrix PF, which is the feature matrix of the exemplary polymer graph in FIG. 3 can also have additional attribute information in column 3, as illustrated in the example below, and this can be represented as an attribute value representing whether each node is an attach node. For example, the polymer node feature matrix PNF below may have attach node attribute information in the fourth column, as illustrated below, in addition to the monomer node feature matrix NF containing three attribute information of the previous four nodes. In the example of FIG. 3, nodes 1 and 4 are attach nodes attached to adjacent repeat unit structures, and thus they have a value of '1', and other nodes are represented as having a value of '0' in column 4.

$$\text{polymer node feature matrix PNF} = \begin{bmatrix} 0 & 1 & 0 & 1 \\ 1 & 0 & 1 & 0 \\ 0 & 1 & 0 & 0 \\ 0 & 1 & 0 & 1 \end{bmatrix}$$

This can be achieved through a polymer node variable setting step of setting respective atoms of the monomer that forms the selected polymer and the attach node, which is the point to which the monomers are repeatedly attached, as a polymer node variable, and a polymer graph node data allocation step of allocating the attach node to be attached to the set polymer node variable and allocating the node attribute value to each node. The node attribute values allocated to the nodes may include atomic number, hybridization (SP3, SP2, etc.), number of hydrogens, number of electrons, ring status, etc., and, in particular, in an embodiment of the present invention, may have an attach node attribute value, which is an attribute value of whether or not the node is attached to an adjacent repeat unit structure.

In another embodiment of the present invention, attaching node data can be represented as an edge feature matrix. For example, instead of representing whether it is an attach node with the node attribute value like the previous polymer node feature matrix or together with it, an attribute value representing whether or not it is an attach edge is represented in the edge feature matrix. In the example below representing (b) of FIG. 3, it can be seen that data of rows 4 and 5 have been added in order to contain information of the edges connecting attach nodes 1 and 4, that is, edges 5_1 and 4_6 in (b) of FIG. 3, and an attribute information column representing whether or not it is an attach edge has been added in column 4.

$$\text{polymer edge feature matrix PEF} = \begin{bmatrix} 1 & 0 & 0 & 0 \\ 0 & 1 & 1 & 0 \\ 0 & 0 & 0 & 0 \\ 0 & 0 & 0 & 1 \\ 0 & 0 & 0 & 1 \end{bmatrix}$$

That is, according to the present invention, the method for representing the structure of a polymer in a graph and converting it into data that can be used on a computer can be selected from one or more of the methods below.

① In the adjacency matrix representing the connection relationship between respective nodes of a repeat unit structure composed of at least one monomer, the component of the 'attach node' attached to the adjacent repeat unit structure is allocated as the 'attach' attribute.

② The node attribute matrix representing the attribute of each node of the repeat unit structure is assigned a 'attach status' attribute field, and 'attach' is allocated to the 'attach status' attribute field of the 'attach node'.

③ Edges connected to the attach nodes are added to the edge attribute matrix of the repeat unit structure as attach edges, the edge attributes are assigned an 'attach status' attribute field, and 'attach' is allocated to the corresponding field of the attach edge.

2.2. Method for converting chemical structure of polymer into data that can be processed by computer

In the present invention, in predicting the properties of the polymer material by training the artificial neural network model described later and using the trained artificial neural network, the chemical structure of the polymer material should be converted into data so that it can be processed by a computer, and it is converted into data according to the method of the present invention as follows.

(1) Repeat unit structure information acquisition step

This is a step of acquiring information of a repeat unit structure, which is a unit in which monomers are repeated to form a polymer. In order to convert attaching node data into data in the repeat unit structure, which is one of the characteristics of the present invention, information of the repeat unit structure forming the polymer is acquired. This is the step of checking whether the repeat unit structure that forms the polymer by being repeated is repeated as a single monomer or a combination of single monomers using the repeat unit structure information, and describing the corresponding repeat unit structure in a graphical representation. The repeat unit structure may be a single monomer as illustrated in (a) of FIG. 2 or may be a combination in which two monomers are attached as illustrated in (b) of FIG. 2. In a case such as (b) of FIG. 2, an adjacent node already becomes the attach point.

(2) Polymer graph node attach data allocation step

The method for processing polymer graph data in the present invention includes a process of setting the interconnection relationship between nodes representing respective atoms constituting the repeat unit structure of the polymer selected for analysis and the attach nodes attached to the attach point, which is the point to which the repeat unit structure is repeatedly attached, among the nodes as variables.

The attribute value of the interconnection relationship of the nodes and the attach nodes is allocated to the polymer node attach variable set in this way. In allocating the attribute value of each connection relationship to the polymer node attach variable, the polymer adjacency matrix, as exemplified above can be utilized.

(3) Polymer graph node attribute information allocation step

The polymer graph data processing method of the present invention may also include a polymer node attribute variable setting step of setting attribute information of the node corresponding to constituent atoms of the repeat unit structure forming the selected polymer and attribute information of the attach node attached to the attach point, which is the point to which the repeat unit structure is repeatedly attached, among the nodes as a polymer node attribute variable.

The polymer graph data processing method further includes a polymer graph node attribute information allocation step of allocating attribute values of the nodes and attach nodes to the set polymer node attribute variables in this way. The node attribute information has an attribute value that indicates whether the node is an attached node or not, and, as node attribute information of the attach node, unlike the nodes that are not attach nodes, and is assigned an attach node attribute value indicating that the node is attached to the attach node of a neighboring repeat unit structure within the polymer.

(4) Polymer graph edge attribute information allocation step

The polymer graph data processing method of the present invention may further include a polymer edge attribute variable setting step of setting information of an edge interconnecting nodes corresponding to respective atoms constituting the repeat unit structure forming the selected polymer and an attach edge connecting at least one of the nodes and the attach node as a polymer edge attribute variable. The attach edge is an edge that connects an attach node to another node.

The polymer graph data processing method of the present invention further includes a polymer graph edge attribute information allocation step of allocating the attribute value of the edges and attached edges to the set polymer edge attribute variable, and the attach edge is assigned an attach edge attribute value, which means that it connects the attach node to another node, as an edge attribute variable thereof.

<Examples and Comparative examples>

An example of representing polymer graph data according to the present invention described above will be described.

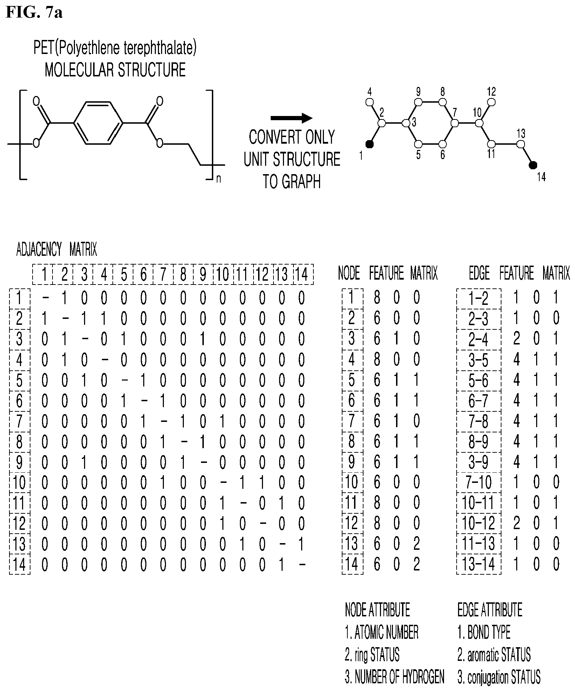

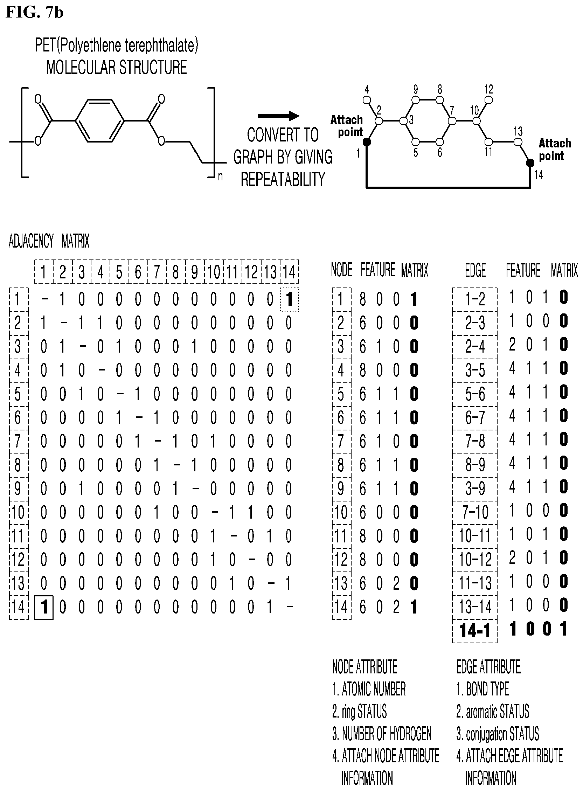

FIG. 7a illustrates a method for representing the molecular structure of polyethylene terephthalate (PET), as an example of a polymer, as graph data according to the prior art, and FIG. 7b illustrates a method for representing polymer graph data according to the present invention.

According to the conventional method, only the monomer structure of the polymer is converted into a graph, as illustrated in FIG. 7a. In this case, an atomic number, a ring status, and the number of hydrogens were used as the node attribute values of the node feature matrix. The edge feature matrix has a bonding type, an aromatic status, and a conjugation status as edge attribute values.

In contrast, the graph data representation method according to the present invention, as illustrated in FIG. 7b, additionally has attach node attribute information as the node attribute value of the node feature matrix, and in the example, node 1 and node 14 are connected to each other, and thus each of nodes 1 and 14 has T 1' as the attach node attribute information value. The edge feature matrix has edge attribute values in addition to attach edge attribute information, and attach edge "14-1" has attribute information '1' as an attach edge.

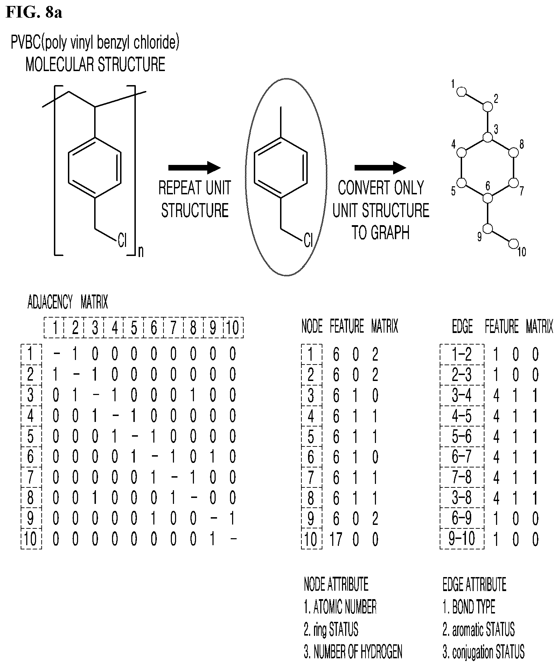

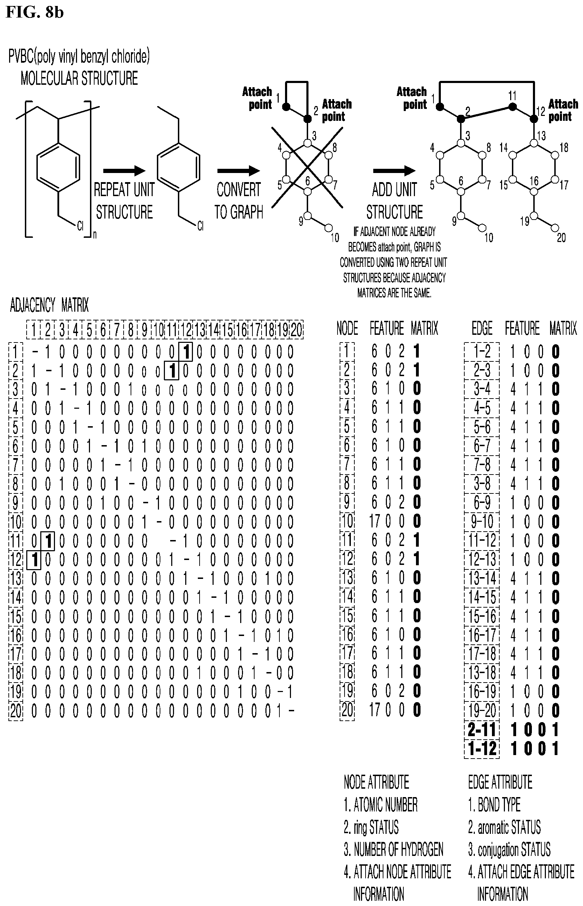

FIG. 8a and 8b illustrates an example of graph informatization of the molecular structure of poly vinyl benzyl chloride (PVBC) as another polymer.

FIG. 8a illustrates the adjacency matrix, the node feature matrix, and the edge feature matrix represented in a method according to the prior art.

FIG. 8b illustrates an example of graph informatization of the molecular structure of PVBC using the method according to the present invention. In FIG. 8b, compared to the method according to the prior art of FIG. 8a, the node feature matrix additionally has attach node attribute information, nodes 1, 2, 11, and 12 become attach nodes in the example, and thus the node attribute information value of these nodes is set to '1'. Like PVBC, when a node already adjacent to a repeat unit structure becomes an attach point, it is converted into graph data using a repeat unit structure in which two monomers are attached. This is the same case as described in relation to (b) of FIG. 2. As illustrated, the edge feature matrix additionally includes attach edge attribute information, and the attach edge attribute information of each of attach edges "2-11" and "1-12" is set to '1'.

3. Method for analyzing polymer properties using graphical representation of polymer according to the present invention

Hereinafter, a method for processing polymer data in an artificial intelligence neural network will be described, according to the polymer graph representation method of the present invention described above.

The present invention provides a method for constructing an artificial neural network model that analyzes predetermined properties of a polymer by applying the polymer representation method described above, and producing predetermined properties of the polymer by performing machine learning on the artificial neural network model.

3.1. Method for generating polymer property analysis model

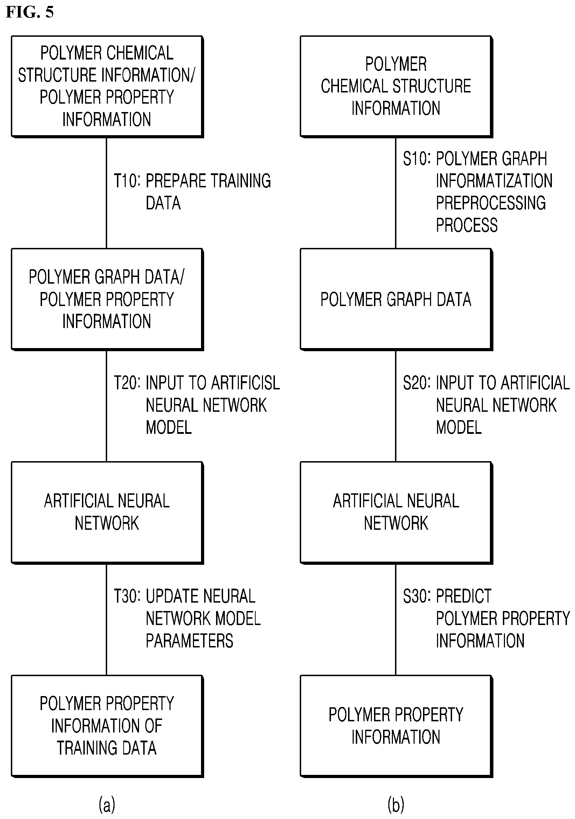

The (a) of FIG. 5 illustrates a method for generating a polymer property analysis model according to the present invention. Based on this, according to the graph data representation method of the present invention, a method for generating a computer-implemented polymer property analysis model that predicts predetermined property information of a selected polymer from the chemical structure of the selected polymer will be described.

(1) Training data preparation process (T10)

First, a training data preparation process of converting the chemical structure information of the learning polymer into the polymer graph data of the present invention in the manner described above from basic data consisting of the chemical structure information of a large number of learning polymers for which predetermined property information is known and the property feature information of the corresponding polymers (T10). This corresponds to a data preprocessing process of the basic data. The training data prepared in this way may be composed of multiple data sets with {learning polymer graph data, known property information of the polymer} as one set. Here, the known property information of the polymer means an actual measured value, actual value, or specified property information of the polymer property information.

(2) Artificial neural network construction and training data input step (T20)

Next, an artificial neural network model to be trained using training data is constructed. As the artificial neural network model in the present invention, a known artificial neural network model is used, and in particular, artificial neural network models of message passing neural network (MPNN) or graph neural network GNN based on message passing can be applied.

For example, when applying GNN, when explaining the case of supervised learning, polymer training data consisting of the sets of {polymer graph data, predetermined property information of the corresponding polymer} configured previously is input to the GNN neural network model. The predetermined property information of the polymer includes, for example, the refractive index, glass transition temperature, and density of the polymer.

In this case, the polymer graph data is input to the neural network model as an input, and the predetermined property information of the corresponding polymer is input as a true value (labeled data) of an output value. In this case, the input polymer graph data includes at least one of the polymer graph node attach data, polymer graph node attribute information, and polymer graph edge attribute information described above, and may be input by being embedded in the polymer adjacency matrix and a polymer node/edge feature matrix.

(3) Training step of artificial neural network model (T30)

This is the process of training a neural network model by updating parameters of the neural network model based on the input polymer graph data and predetermined property information of the polymer. The training process of the neural network model follows the known neural network model training process. For example, the parameters of the artificial neural network are updated so that an error function defined as a difference between a predicted value of the property information of the polymer produced from the neural network model by receiving the polymer graph data and the predetermined property information of the corresponding polymer among the training data is minimized.

3.2. Polymer property analysis method

This is a method for predicting the property information of the selected polymer by inputting the graph data of the selected polymer into the artificial neural network generated according to the method of generating the polymer property analysis model of the present invention described above. Description will be made with reference to FIG. 5(b).

(1) Polymer graph informatization preprocessing process (S10)

A preprocessing process of obtaining polymer graph data from the chemical structure of the selected polymer for which property information is to be predicted is performed. As described above, the polymer graph data includes the attach node and attach edge 40 information between repeating monomers in addition to node and edge data of the monomers constituting the polymer. That is, the polymer graph data is preprocessed to include at least one of the polymer graph node attach data, polymer graph node attribute information, and polymer graph edge attribute information described above.

(2) Polymer graph data input step (S20)

This is the step of inputting polymer graph data based on the chemical structure of the selected polymer into the previously trained neural network model. In this case, the input polymer graph data includes at least one of the polymer graph node attach data, polymer graph node attribute information, and polymer graph edge attribute information described above, and may be input by being embedded in the polymer adjacency matrix and polymer node/edge feature matrix described above.

(3) Polymer property information prediction step (S30)

This is the step of producing and outputting the property information of the selected polymer from the input polymer graph data of the selected polymer using the trained artificial neural network model.

4. Polymer property information prediction system 300 according to the present invention

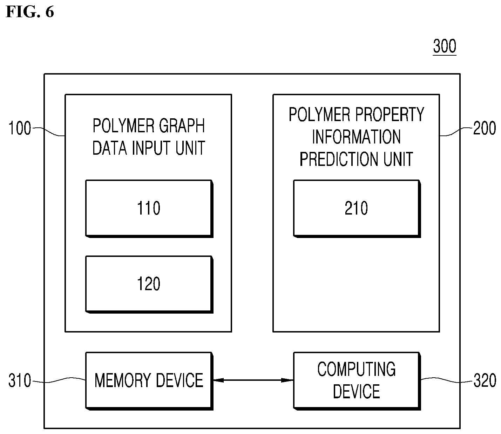

With reference to FIG. 6, the polymer property information prediction system according to the present invention will be described.

(1) Polymer graph data input unit 100

The polymer graph data input unit 100 is configured to receive input by converting the chemical structure of the selected polymer into polymer graph data that describes it as graphic data.

The polymer graph data input unit 100 includes a polymer graph data acquisition unit 110 that acquires the polymer graph data from the chemical structure of the selected polymer for which property information is to be predicted.

The polymer graph data acquisition unit 110 generates polymer graph data by allocating and processing the attach node and attach edge data between repeating repeat unit structures to respective variables, in addition to the node and edge data of the monomers that make up the polymer described above. That is, the polymer graph data is a data set that includes at least one of the polymer graph node attach data, polymer graph node attribute information, and polymer graph edge attribute information described above, and the polymer graph attach data is information representing the interconnection relationship between the nodes corresponding to respective atoms constituting the repeat unit structure forming the polymer and the attach nodes attached to an attach point, which is a point to which the repeat unit structure is repeatedly attached, among the nodes.

The polymer graph input unit 100 can be additionally provided with a matrix conversion unit 120 that generates and inputs a polymer adjacency matrix including the interconnection relationship between two or more nodes representing respective atoms that makes up a single molecule of a polymer and information of connection relationship between the attach node and the nodes and a polymer edge attribute matrix including attribute information of one or more edges representing the bond between the respective nodes and an attach edge representing the connection between the nodes and the attach node, in inputting the polymer graph data into the artificial neural network model unit 200, which will be described later.

In this case, the polymer graph data , as described above, may include at least one of the polymer graph node attach data, which is information representing the interconnection relationship between the nodes corresponding to respective atoms constituting the repeat unit structure forming the polymer and the attach nodes attached to an attach point, which is a point to which the repeat unit structure is repeatedly attached, among the nodes, the polymer node attribute information, which is information representing attribute information of the node corresponding to respective atoms constituting the repeat unit structure forming the polymer and the attribute of the attach node attached to an attach point, which is a point to which the repeat unit structure is repeatedly attached, among the nodes, and the polymer edge attribute information, which is information representing the attributes of the edge connecting the nodes corresponding to respective atoms constituting the repeat unit structure forming the polymer and the attach edge connecting at least one of the nodes and the attach node.

(2) Polymer property information prediction unit (200)

The polymer property information prediction unit 200 is configured to include an artificial neural network 210 that is trained to predict and produce predetermined property information of the polymer from the polymer graph data.

The artificial neural network is configured to receive the polymer graph data described above and output a predicted value of predetermined property information of the polymer, produces the predicted value of predetermined property information of the polymer from the polymer graph data of the predetermined learning polymer, and is machine learned by updating the parameters of the neural network according to the procedure described above so that the error function defined as the difference from the label value of the property information is minimized.

(3) Computer device

The polymer property information prediction system 300 according to the present invention may be configured with a single computing system or a computer device in which multiple computing systems are combined in a network. In the computer device, the polymer graph data input unit 100 and the polymer property information prediction unit 200 described above may be configured with a memory device 310 and a computing device 320 that constitute the computer device. That is, the computer device 300 is configured to include a memory device 310 and the computing device 320, and the polymer graph data input unit 100 and the polymer property information prediction unit 200 described above are configured by occupying predetermined functions and parts of the memory device 310 and the computing device 320.

The polymer graph data input unit 100 and the polymer property information prediction unit 200 may be configured to include a computer algorithm that is stored in the memory device 310 of the computer device and performs each procedure by being read by the computing device 320 in generating and processing the polymer graph data, or may be configured with the computer algorithm.

The reference numerals used in the description of the present invention are as follows.

10: Node 20: Edge
30: Attach point 40: Attach edge
100: Polymer graph data input unit
110: Polymer graph data acquisition unit
120: Matrix conversion unit
200: Polymer property information prediction unit
210: Artificial neural network
310: Memory device 320: Computing device

**Claims**

1. A data processing method of polymer chemical structure, in a method for converting a chemical structure data of a polymer into data so as to be able to be processed by a computer, comprising:

   a polymer node attach variable setting step of setting an interconnection relationship between nodes representing respective atoms constituting a repeat unit structure of a selected polymer and attach nodes attached to an attach point, which is a point to which the repeat unit structure is repeatedly attached, among the nodes as a polymer node attach variable; and
   a polymer graph node attach data allocation step of allocating an attribute value of the interconnection relationship between the nodes and the attach nodes to the set polymer node attach variable.

2. The data processing method of claim 1, further comprising:

   a polymer node attribute variable setting step of setting attribute information of the node corresponding to constituent atoms of the repeat unit structure forming the selected polymer and information of the attach node attached to the attach point, which is the point to which the repeat unit structure is repeatedly attached, among the nodes as a polymer node attribute variable; and
   a polymer graph node attribute information allocation step of allocating an attribute value of the nodes and attach nodes to the set polymer node attribute variable.

3. The data processing method of claim 2, further comprising:

   a polymer edge attribute variable setting step of setting information of an edge interconnecting nodes corresponding to respective atoms constituting the repeat unit structure forming the selected polymer and an attach edge connecting at least one of the nodes and the attach node as a polymer edge attribute variable; and
   a polymer graph edge attribute information allocation step of allocating attribute values of the edges and attached edges to the set polymer edge attribute variable.

4. A generation method of polymer property analysis model, as a method for generating a computer-implemented model that graphically describes a chemical structure of a selected polymer and analyzes predetermined properties of the polymer, comprising:

   a basic data acquisition step of acquiring basic data consisting of chemical structure information of a plurality of learning polymers and known values of predetermined property information of the corresponding polymers;
   a data pre-processing process of converting the chemical structure information of the corresponding learning polymer among the basic data into polymer graph data;
   a polymer property information prediction artificial neural network construction step of constructing a polymer property information prediction artificial neural network so as to receive the polymer graph data and output a predetermined property information prediction value of the polymer;
   a training data input step of inputting the polymer graph data of the learning polymer and the predetermined property information of the learning polymer into the polymer property information prediction artificial neural network; and
   a learning step of the polymer property information prediction artificial neural network for updating parameters of the polymer property information prediction artificial neural network, based on a comparison result between the predetermined property information prediction value of the learning polymer output by the polymer property information prediction artificial neural network and the known value of the predetermined property information of the learning polymer.

5. The generation method of claim 4, wherein
the polymer graph data comprises at least one of

polymer graph node attach data which is information representing an interconnection relationship between nodes corresponding to respective atoms constituting a repeat unit structure forming the polymer and attach nodes attached to an attach point, which is a point to which the repeat unit structure is repeatedly attached, among the nodes,
polymer node attribute information, which is information representing attribute information of the node corresponding to respective atoms constituting the repeat unit structure forming the polymer, and representing an attribute of the attach nodes attached to an attach point, which is a point to which the repeat unit structure is repeatedly attached, among the nodes, and
polymer edge attribute information, which is information representing the attributes of an edge interconnecting the nodes corresponding to respective atoms constituting the repeat unit structure forming the polymer, and an attach edge connecting at least one of the nodes and the attach node.

6. A computer-implemented method for graphically describing a chemical structure of a selected polymer and analyzing predetermined properties of the polymer, the computer-implemented method comprising:

a polymer property information production artificial neural network construction step of generating an artificial neural network that is subjected to machine learning so as to predict predetermined properties of the selected polymer based at least in part on chemical structure data related to the polymer;
a polymer graph data acquisition step of acquiring polymer graph data that describes the chemical structure of the selected polymer as graphic data;
a polymer graph input step of providing the acquired polymer graph data as an input to the graph neural network subjected to machine learning; and
a step of receiving prediction data describing the predetermined properties of the selected polymer as an output of the graph neural network subjected to machine learning, wherein
the polymer graph data that describes the chemical structure of the selected polymer as graphic data comprises at least one of

polymer graph node attach data which is information representing an interconnection relationship between nodes corresponding to respective atoms constituting a repeat unit structure forming the polymer and attach nodes attached to an attach point, which is a point to which the repeat unit structure is repeatedly attached, among the nodes,
polymer node attribute information, which is information representing attribute information of the node corresponding to respective atoms constituting the repeat unit structure forming the polymer, and an attribute of the attach node attached to the attach point, which is a point to which the repeat unit structure is repeatedly attached, among the nodes, and
polymer edge attribute information, which is information representing attributes of an edge interconnecting nodes corresponding to respective atoms constituting the repeat unit structure forming the polymer, and an attach edge connecting at least one of the nodes and the attach node.

7. The computer-implemented method of claim 6, wherein

the step of providing the acquired polymer graph data as the input to the graph neural network subjected to machine learning comprises

a polymer adjacency matrix construction step of constructing an adjacency matrix representing an interconnection relationship between two or more nodes representing respective atoms constituting the single molecule, and
a polymer adjacency matrix input step of inputting the constructed adjacency matrix into the graph neural network, and

the polymer adjacency matrix further comprises connection relationship information between the attach node and the nodes.

8. The computer-implemented method of claim 7, wherein

the step of providing the acquired polymer graph data as the input to the graph neural network subjected to machine learning comprises
a polymer edge attribute matrix input step of generating and inputting a polymer edge attribute matrix including attribute information of one or more edges representing a bond between the respective nodes and an attach edge representing a connection between the nodes and the attach node.

9. The computer-implemented method of claim 6, wherein

a step of acquiring, by one or more computing devices, an artificial neural network that is subjected to machine learning so as to predict predetermined properties of the polymer based at least in part on a chemical structure data related to the polymer comprises

a step of acquiring, by the one or more computing devices, training data including chemical structures of a plurality of exemplary polymers and predetermined feature label values that describe predetermined properties of the exemplary polymers having the chemical structures of the exemplary polymers, and
a training step of inputting graph data graphically describing the chemical structures of the exemplary polymers by acquiring the graph information and training the graph neural network so as to output a predetermined feature label describing the predetermined properties of the exemplary polymers output by the graph neural network.

10. The computer-implemented method of claim 9, wherein

the graph data graphically describing the chemical structures of the exemplary polymers is composed of

two or more node data representing respective atoms constituting a single molecule forming the polymer,
one or more edge data representing a bond between the respective atoms, attaching node data which is a point to which the single molecule is repeatedly attached, and
attach edge data representing a connection between the atom and the attach node.

11. A system for graphically describing a chemical structure of a selected polymer and analyzing predetermined properties of the polymer, comprising:

a polymer graph data input unit that receives polymer graph information by representing the chemical structure of the selected polymer as polymer graph data; and
a polymer property information prediction unit that outputs a predicted value of predetermined property information of the selected polymer from the polymer graph information, wherein
the polymer graph data comprises
polymer graph node attach data which is information representing an interconnection relationship between nodes corresponding to respective atoms constituting a repeat unit structure forming the polymer and attach nodes attached to an attach point, which is a point to which the repeat unit structure is repeatedly attached, among the nodes, and
the polymer property information prediction unit comprises
an artificial neural network trained to output a predicted value of predetermined polymer property information from the polymer graph data including the polymer graph node attach data.

12. The system of claim 11, wherein

the polymer graph data input unit
converts the chemical structure of the polymer into the polymer graph data, and
the polymer graph data further comprises at least one of

polymer node attribute information which is information representing attribute information of the node corresponding to respective atoms constituting the repeat unit structure forming the polymer and representing an attribute of the attach nodes attached to an attach point, which is a point to which the repeat unit structure is repeatedly attached, among the nodes, and
polymer edge attribute information which is information representing the attributes of an edge interconnecting the nodes corresponding to respective atoms constituting the repeat unit structure forming the polymer

and an attach edge connecting at least one of the nodes and the attach node.

**FIG. 1**

monomer A

polymer A

(a)

monomer B

polymer B

(b)

**FIG. 2**

Polymer graph representation

(a)

(b)

FIG. 3

(a)

(b)

**FIG. 4**

**FIG. 5**

POLYMER CHEMICAL
STRUCTURE INFORMATION/
POLYMER PROPERTY
INFORMATION

T10: PREPARE TRAINING
DATA

POLYMER GRAPH DATA/
POLYMER PROPERTY
INFORMATION

T20: INPUT TO ARTIFICISL
NEURAL NETWORK
MODEL

ARTIFICIAL NEURAL
NETWORK

T30: UPDATE NEURAL
NETWORK MODEL
PARAMETERS

POLYMER PROPERTY
INFORMATION OF
TRAINING DATA

(a)

POLYMER
CHEMICAL STRUCTURE
INFORMATION

S10: POLYMER GRAPH
INFORMATIZATION
PREPROCESSING
PROCESS

POLYMER GRAPH DATA

S20: INPUT TO ARTIFICIAL
NEURAL NETWORK
MODEL

ARTIFICIAL NEURAL
NETWORK

S30: PREDICT
POLYMER PROPERTY
INFORMATION

POLYMER PROPERTY
INFORMATION

(b)

**FIG. 6**

300

| | |
|---|---|
| **POLYMER GRAPH DATA INPUT UNIT** 100 | **POLYMER PROPERTY INFORMATION PREDICTION UNIT** 200 |
| 110 | 210 |
| 120 | |

| | |
|---|---|
| 310 MEMORY DEVICE | ◄——► COMPUTING DEVICE 320 |

**FIG. 7a**

PET(Polyethlene terephthalate)
MOLECULAR STRUCTURE

CONVERT ONLY
UNIT STRUCTURE
TO GRAPH

ADJACENCY MATRIX

|    | 1 | 2 | 3 | 4 | 5 | 6 | 7 | 8 | 9 | 10 | 11 | 12 | 13 | 14 |
|----|---|---|---|---|---|---|---|---|---|----|----|----|----|----|
| 1  | – | 1 | 0 | 0 | 0 | 0 | 0 | 0 | 0 | 0  | 0  | 0  | 0  | 0  |
| 2  | 1 | – | 1 | 1 | 0 | 0 | 0 | 0 | 0 | 0  | 0  | 0  | 0  | 0  |
| 3  | 0 | 1 | – | 0 | 1 | 0 | 0 | 0 | 1 | 0  | 0  | 0  | 0  | 0  |
| 4  | 0 | 1 | 0 | – | 0 | 0 | 0 | 0 | 0 | 0  | 0  | 0  | 0  | 0  |
| 5  | 0 | 0 | 1 | 0 | – | 1 | 0 | 0 | 0 | 0  | 0  | 0  | 0  | 0  |
| 6  | 0 | 0 | 0 | 0 | 1 | – | 1 | 0 | 0 | 0  | 0  | 0  | 0  | 0  |
| 7  | 0 | 0 | 0 | 0 | 0 | 1 | – | 1 | 0 | 1  | 0  | 0  | 0  | 0  |
| 8  | 0 | 0 | 0 | 0 | 0 | 0 | 1 | – | 1 | 0  | 0  | 0  | 0  | 0  |
| 9  | 0 | 0 | 1 | 0 | 0 | 0 | 0 | 1 | – | 0  | 0  | 0  | 0  | 0  |
| 10 | 0 | 0 | 0 | 0 | 0 | 0 | 1 | 0 | 0 | –  | 1  | 1  | 0  | 0  |
| 11 | 0 | 0 | 0 | 0 | 0 | 0 | 0 | 0 | 0 | 1  | –  | 0  | 1  | 0  |
| 12 | 0 | 0 | 0 | 0 | 0 | 0 | 0 | 0 | 0 | 1  | 0  | –  | 0  | 0  |
| 13 | 0 | 0 | 0 | 0 | 0 | 0 | 0 | 0 | 0 | 0  | 1  | 0  | –  | 1  |
| 14 | 0 | 0 | 0 | 0 | 0 | 0 | 0 | 0 | 0 | 0  | 0  | 0  | 1  | –  |

NODE FEATURE MATRIX

|    |   |   |   |
|----|---|---|---|
| 1  | 8 | 0 | 0 |
| 2  | 6 | 0 | 0 |
| 3  | 6 | 1 | 0 |
| 4  | 8 | 0 | 0 |
| 5  | 6 | 1 | 1 |
| 6  | 6 | 1 | 1 |
| 7  | 6 | 1 | 0 |
| 8  | 6 | 1 | 1 |
| 9  | 6 | 1 | 1 |
| 10 | 6 | 0 | 0 |
| 11 | 8 | 0 | 0 |
| 12 | 8 | 0 | 0 |
| 13 | 6 | 0 | 2 |
| 14 | 6 | 0 | 2 |

EDGE FEATURE MATRIX

|       |   |   |   |
|-------|---|---|---|
| 1-2   | 1 | 0 | 1 |
| 2-3   | 1 | 0 | 0 |
| 2-4   | 2 | 0 | 1 |
| 3-5   | 4 | 1 | 1 |
| 5-6   | 4 | 1 | 1 |
| 6-7   | 4 | 1 | 1 |
| 7-8   | 4 | 1 | 1 |
| 8-9   | 4 | 1 | 1 |
| 3-9   | 4 | 1 | 1 |
| 7-10  | 1 | 0 | 0 |
| 10-11 | 1 | 0 | 1 |
| 10-12 | 2 | 0 | 1 |
| 11-13 | 1 | 0 | 0 |
| 13-14 | 1 | 0 | 0 |

NODE ATTRIBUTE
1. ATOMIC NUMBER
2. ring STATUS
3. NUMBER OF HYDROGEN

EDGE ATTRIBUTE
1. BOND TYPE
2. aromatic STATUS
3. conjugation STATUS

## FIG. 7b

PET(Polyethlene terephthalate)
MOLECULAR STRUCTURE

CONVERT TO
GRAPH BY GIVING
REPEATABILITY

ADJACENCY MATRIX

|    | 1 | 2 | 3 | 4 | 5 | 6 | 7 | 8 | 9 | 10 | 11 | 12 | 13 | 14 |
|----|---|---|---|---|---|---|---|---|---|----|----|----|----|----|
| 1  | - | 1 | 0 | 0 | 0 | 0 | 0 | 0 | 0 | 0  | 0  | 0  | 0  | **1** |
| 2  | 1 | - | 1 | 1 | 0 | 0 | 0 | 0 | 0 | 0  | 0  | 0  | 0  | 0  |
| 3  | 0 | 1 | - | 0 | 1 | 0 | 0 | 0 | 1 | 0  | 0  | 0  | 0  | 0  |
| 4  | 0 | 1 | 0 | - | 0 | 0 | 0 | 0 | 0 | 0  | 0  | 0  | 0  | 0  |
| 5  | 0 | 0 | 1 | 0 | - | 1 | 0 | 0 | 0 | 0  | 0  | 0  | 0  | 0  |
| 6  | 0 | 0 | 0 | 0 | 1 | - | 1 | 0 | 0 | 0  | 0  | 0  | 0  | 0  |
| 7  | 0 | 0 | 0 | 0 | 0 | 1 | - | 1 | 0 | 1  | 0  | 0  | 0  | 0  |
| 8  | 0 | 0 | 0 | 0 | 0 | 0 | 1 | - | 1 | 0  | 0  | 0  | 0  | 0  |
| 9  | 0 | 0 | 1 | 0 | 0 | 0 | 0 | 1 | - | 0  | 0  | 0  | 0  | 0  |
| 10 | 0 | 0 | 0 | 0 | 0 | 0 | 1 | 0 | 0 | -  | 1  | 1  | 0  | 0  |
| 11 | 0 | 0 | 0 | 0 | 0 | 0 | 0 | 0 | 0 | 1  | -  | 0  | 1  | 0  |
| 12 | 0 | 0 | 0 | 0 | 0 | 0 | 0 | 0 | 0 | 1  | 0  | -  | 0  | 0  |
| 13 | 0 | 0 | 0 | 0 | 0 | 0 | 0 | 0 | 0 | 0  | 1  | 0  | -  | 1  |
| 14 | **1** | 0 | 0 | 0 | 0 | 0 | 0 | 0 | 0 | 0  | 0  | 0  | 1  | -  |

NODE FEATURE MATRIX

| | | | | |
|----|---|---|---|---|
| 1  | 8 | 0 | 0 | **1** |
| 2  | 6 | 0 | 0 | **0** |
| 3  | 6 | 1 | 0 | **0** |
| 4  | 8 | 0 | 0 | **0** |
| 5  | 6 | 1 | 1 | **0** |
| 6  | 6 | 1 | 1 | **0** |
| 7  | 6 | 1 | 0 | **0** |
| 8  | 6 | 1 | 1 | **0** |
| 9  | 6 | 1 | 1 | **0** |
| 10 | 6 | 0 | 0 | **0** |
| 11 | 8 | 0 | 0 | **0** |
| 12 | 8 | 0 | 0 | **0** |
| 13 | 6 | 0 | 2 | **0** |
| 14 | 6 | 0 | 2 | **1** |

EDGE FEATURE MATRIX

| EDGE | | | | |
|------|---|---|---|---|
| 1-2  | 1 | 0 | 1 | **0** |
| 2-3  | 1 | 0 | 0 | **0** |
| 2-4  | 2 | 0 | 1 | **0** |
| 3-5  | 4 | 1 | 1 | **0** |
| 5-6  | 4 | 1 | 1 | **0** |
| 6-7  | 4 | 1 | 1 | **0** |
| 7-8  | 4 | 1 | 1 | **0** |
| 8-9  | 4 | 1 | 1 | **0** |
| 3-9  | 4 | 1 | 1 | **0** |
| 7-10 | 1 | 0 | 0 | **0** |
| 10-11 | 1 | 0 | 1 | **0** |
| 10-12 | 2 | 0 | 1 | **0** |
| 11-13 | 1 | 0 | 0 | **0** |
| 13-14 | 1 | 0 | 0 | **0** |
| **14-1** | **1** | **0** | **0** | **1** |

NODE ATTRIBUTE
1. ATOMIC NUMBER
2. ring STATUS
3. NUMBER OF HYDROGEN
4. ATTACH NODE ATTRIBUTE
   INFORMATION

EDGE ATTRIBUTE
1. BOND TYPE
2. aromatic STATUS
3. conjugation STATUS
4. ATTACH EDGE ATTRIBUTE
   INFORMATION

**FIG. 8a**

PVBC(poly vinyl benzyl chloride)
MOLECULAR STRUCTURE

REPEAT UNIT STRUCTURE

CONVERT ONLY UNIT STRUCTURE TO GRAPH

ADJACENCY MATRIX

|    | 1 | 2 | 3 | 4 | 5 | 6 | 7 | 8 | 9 | 10 |
|----|---|---|---|---|---|---|---|---|---|----|
| 1  | – | 1 | 0 | 0 | 0 | 0 | 0 | 0 | 0 | 0 |
| 2  | 1 | – | 1 | 0 | 0 | 0 | 0 | 0 | 0 | 0 |
| 3  | 0 | 1 | – | 1 | 0 | 0 | 0 | 1 | 0 | 0 |
| 4  | 0 | 0 | 1 | – | 1 | 0 | 0 | 0 | 0 | 0 |
| 5  | 0 | 0 | 0 | 1 | – | 1 | 0 | 0 | 0 | 0 |
| 6  | 0 | 0 | 0 | 0 | 1 | – | 1 | 0 | 1 | 0 |
| 7  | 0 | 0 | 0 | 0 | 0 | 1 | – | 1 | 0 | 0 |
| 8  | 0 | 0 | 1 | 0 | 0 | 0 | 1 | – | 0 | 0 |
| 9  | 0 | 0 | 0 | 0 | 0 | 1 | 0 | 0 | – | 1 |
| 10 | 0 | 0 | 0 | 0 | 0 | 0 | 0 | 0 | 1 | – |

NODE FEATURE MATRIX

|    |    |   |   |
|----|----|---|---|
| 1  | 6  | 0 | 2 |
| 2  | 6  | 0 | 2 |
| 3  | 6  | 1 | 0 |
| 4  | 6  | 1 | 1 |
| 5  | 6  | 1 | 1 |
| 6  | 6  | 1 | 0 |
| 7  | 6  | 1 | 1 |
| 8  | 6  | 1 | 1 |
| 9  | 6  | 0 | 2 |
| 10 | 17 | 0 | 0 |

EDGE FEATURE MATRIX

|      |   |   |   |
|------|---|---|---|
| 1–2  | 1 | 0 | 0 |
| 2–3  | 1 | 0 | 0 |
| 3–4  | 4 | 1 | 1 |
| 4–5  | 4 | 1 | 1 |
| 5–6  | 4 | 1 | 1 |
| 6–7  | 4 | 1 | 1 |
| 7–8  | 4 | 1 | 1 |
| 3–8  | 4 | 1 | 1 |
| 6–9  | 1 | 0 | 0 |
| 9–10 | 1 | 0 | 0 |

NODE ATTRIBUTE
1. ATOMIC NUMBER
2. ring STATUS
3. NUMBER OF HYDROGEN

EDGE ATTRIBUTE
1. BOND TYPE
2. aromatic STATUS
3. conjugation STATUS

## FIG. 8b

PVBC(poly vinyl benzyl chloride)
MOLECULAR STRUCTURE

REPEAT UNIT STRUCTURE → CONVERT TO GRAPH → ADD UNIT STRUCTURE

IF ADJACENT NODE ALREADY BECOMES attach point, GRAPH IS CONVERTED USING TWO REPEAT UNIT STRUCTURES BECAUSE ADJACENCY MATRICES ARE THE SAME.

### ADJACENCY MATRIX

|    | 1 | 2 | 3 | 4 | 5 | 6 | 7 | 8 | 9 | 10 | 11 | 12 | 13 | 14 | 15 | 16 | 17 | 18 | 19 | 20 |
|----|---|---|---|---|---|---|---|---|---|----|----|----|----|----|----|----|----|----|----|----|
| 1  | - | 1 | 0 | 0 | 0 | 0 | 0 | 0 | 0 | 0  | 0  | **1** | 0 | 0 | 0 | 0 | 0 | 0 | 0 | 0 |
| 2  | 1 | - | 1 | 0 | 0 | 0 | 0 | 0 | 0 | 0  | **1** | 0 | 0 | 0 | 0 | 0 | 0 | 0 | 0 | 0 |
| 3  | 0 | 1 | - | 1 | 0 | 0 | 0 | 1 | 0 | 0  | 0  | 0  | 0 | 0 | 0 | 0 | 0 | 0 | 0 | 0 |
| 4  | 0 | 0 | 1 | - | 1 | 0 | 0 | 0 | 0 | 0  | 0  | 0  | 0 | 0 | 0 | 0 | 0 | 0 | 0 | 0 |
| 5  | 0 | 0 | 0 | 1 | - | 1 | 0 | 0 | 0 | 0  | 0  | 0  | 0 | 0 | 0 | 0 | 0 | 0 | 0 | 0 |
| 6  | 0 | 0 | 0 | 0 | 1 | - | 1 | 0 | 1 | 0  | 0  | 0  | 0 | 0 | 0 | 0 | 0 | 0 | 0 | 0 |
| 7  | 0 | 0 | 0 | 0 | 0 | 1 | - | 1 | 0 | 0  | 0  | 0  | 0 | 0 | 0 | 0 | 0 | 0 | 0 | 0 |
| 8  | 0 | 0 | 1 | 0 | 0 | 0 | 1 | - | 0 | 0  | 0  | 0  | 0 | 0 | 0 | 0 | 0 | 0 | 0 | 0 |
| 9  | 0 | 0 | 0 | 0 | 0 | 1 | 0 | 0 | - | 1  | 0  | 0  | 0 | 0 | 0 | 0 | 0 | 0 | 0 | 0 |
| 10 | 0 | 0 | 0 | 0 | 0 | 0 | 0 | 0 | 1 | -  | 0  | 0  | 0 | 0 | 0 | 0 | 0 | 0 | 0 | 0 |
| 11 | 0 | **1** | 0 | 0 | 0 | 0 | 0 | 0 | 0 | 0 | - | 1 | 0 | 0 | 0 | 0 | 0 | 0 | 0 | 0 |
| 12 | **1** | 0 | 0 | 0 | 0 | 0 | 0 | 0 | 0 | 0 | 1 | - | 1 | 0 | 0 | 0 | 0 | 0 | 0 | 0 |
| 13 | 0 | 0 | 0 | 0 | 0 | 0 | 0 | 0 | 0 | 0 | 0 | 1 | - | 1 | 0 | 0 | 0 | 1 | 0 | 0 |
| 14 | 0 | 0 | 0 | 0 | 0 | 0 | 0 | 0 | 0 | 0 | 0 | 0 | 1 | - | 1 | 0 | 0 | 0 | 0 | 0 |
| 15 | 0 | 0 | 0 | 0 | 0 | 0 | 0 | 0 | 0 | 0 | 0 | 0 | 0 | 1 | - | 1 | 0 | 0 | 0 | 0 |
| 16 | 0 | 0 | 0 | 0 | 0 | 0 | 0 | 0 | 0 | 0 | 0 | 0 | 0 | 0 | 1 | - | 1 | 0 | 1 | 0 |
| 17 | 0 | 0 | 0 | 0 | 0 | 0 | 0 | 0 | 0 | 0 | 0 | 0 | 0 | 0 | 0 | 1 | - | 1 | 0 | 0 |
| 18 | 0 | 0 | 0 | 0 | 0 | 0 | 0 | 0 | 0 | 0 | 0 | 0 | 1 | 0 | 0 | 0 | 1 | - | 0 | 0 |
| 19 | 0 | 0 | 0 | 0 | 0 | 0 | 0 | 0 | 0 | 0 | 0 | 0 | 0 | 0 | 0 | 1 | 0 | 0 | - | 1 |
| 20 | 0 | 0 | 0 | 0 | 0 | 0 | 0 | 0 | 0 | 0 | 0 | 0 | 0 | 0 | 0 | 0 | 0 | 0 | 1 | - |

### NODE FEATURE MATRIX

| NODE | | | |
|------|---|---|---|
| 1  | 6  | 0 | 2 | **1** |
| 2  | 6  | 0 | 2 | **1** |
| 3  | 6  | 1 | 0 | **0** |
| 4  | 6  | 1 | 1 | **0** |
| 5  | 6  | 1 | 1 | **0** |
| 6  | 6  | 1 | 0 | **0** |
| 7  | 6  | 1 | 1 | **0** |
| 8  | 6  | 1 | 1 | **0** |
| 9  | 6  | 0 | 2 | **0** |
| 10 | 17 | 0 | 0 | **0** |
| 11 | 6  | 0 | 2 | **1** |
| 12 | 6  | 0 | 2 | **1** |
| 13 | 6  | 1 | 0 | **0** |
| 14 | 6  | 1 | 1 | **0** |
| 15 | 6  | 1 | 1 | **0** |
| 16 | 6  | 1 | 0 | **0** |
| 17 | 6  | 1 | 1 | **0** |
| 18 | 6  | 1 | 1 | **0** |
| 19 | 6  | 0 | 2 | **0** |
| 20 | 17 | 0 | 0 | **0** |

### EDGE FEATURE MATRIX

| EDGE | | | |
|-------|---|---|---|
| 1-2   | 1 | 0 | 0 | **0** |
| 2-3   | 1 | 0 | 0 | **0** |
| 3-4   | 4 | 1 | 1 | **0** |
| 4-5   | 4 | 1 | 1 | **0** |
| 5-6   | 4 | 1 | 1 | **0** |
| 6-7   | 4 | 1 | 1 | **0** |
| 7-8   | 4 | 1 | 1 | **0** |
| 3-8   | 4 | 1 | 1 | **0** |
| 6-9   | 1 | 0 | 0 | **0** |
| 9-10  | 1 | 0 | 0 | **0** |
| 11-12 | 1 | 0 | 0 | **0** |
| 12-13 | 1 | 0 | 0 | **0** |
| 13-14 | 4 | 1 | 1 | **0** |
| 14-15 | 4 | 1 | 1 | **0** |
| 15-16 | 4 | 1 | 1 | **0** |
| 16-17 | 4 | 1 | 1 | **0** |
| 17-18 | 4 | 1 | 1 | **0** |
| 13-18 | 4 | 1 | 1 | **0** |
| 16-19 | 1 | 0 | 0 | **0** |
| 19-20 | 1 | 0 | 0 | **0** |
| **2-11** | **1** | **0** | **0** | **1** |
| **1-12** | **1** | **0** | **0** | **1** |

NODE ATTRIBUTE
1. ATOMIC NUMBER
2. ring STATUS
3. NUMBER OF HYDROGEN
4. ATTACH NODE ATTRIBUTE INFORMATION

EDGE ATTRIBUTE
1. BOND TYPE
2. aromatic STATUS
3. conjugation STATUS
4. ATTACH EDGE ATTRIBUTE INFORMATION

## INTERNATIONAL SEARCH REPORT

| International application No. |
| --- |
| **PCT/KR2023/015926** |

**A.    CLASSIFICATION OF SUBJECT MATTER**

**G16C 20/80**(2019.01)i; **G16C 20/90**(2019.01)i; **G16C 20/30**(2019.01)i; **G16C 20/70**(2019.01)i; **G06N 3/08**(2006.01)i

According to International Patent Classification (IPC) or to both national classification and IPC

**B.    FIELDS SEARCHED**

Minimum documentation searched (classification system followed by classification symbols)

G16C 20/80(2019.01); G06N 20/00(2019.01); G06N 3/04(2006.01); G06N 3/08(2006.01); G16C 20/10(2019.01); G16C 20/30(2019.01); G16C 20/50(2019.01); G16C 20/62(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 신경망(neural network), 노드(node), 고분자(polymer), 변수(variable), 그래프(graph), 구조(structure), 데이터(data)

**C.    DOCUMENTS CONSIDERED TO BE RELEVANT**

| Category* | Citation of document, with indication, where appropriate, of the relevant passages | Relevant to claim No. |
| --- | --- | --- |
| Y | JP 2020-009203 A (KWANSEI GAKUIN UNIV.) 16 January 2020 (2020-01-16)<br>See claim 1; paragraphs [0011]-[0013], [0018], [0021], [0028]-[0029] and [0036]; and figures 2 and 3. | 1-12 |
| Y | ROY, N. K. et al. Polymer Property Prediction and Optimization Using Neural Networks. IEEE TRANSACTIONS ON NEURAL NETWORKS. 2006, vol. 17, no. 4, pp. 1001-1014.<br>See pages 1001, 1003 and 1007-1008. | 1-12 |
| A | KR 10-2022-0112692 A (LG CHEM, LTD.) 11 August 2022 (2022-08-11)<br>See entire document. | 1-12 |
| A | KR 10-2021-0047262 A (STANDIGM INC.) 29 April 2021 (2021-04-29)<br>See entire document. | 1-12 |
| A | JP 2021-081769 A (DNA:KK) 27 May 2021 (2021-05-27)<br>See entire document. | 1-12 |

☑ Further documents are listed in the continuation of Box C.        ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **05 February 2024** | **05 February 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/015926** |

**C.     DOCUMENTS CONSIDERED TO BE RELEVANT**

| Category* | Citation of document, with indication, where appropriate, of the relevant passages | Relevant to claim No. |
| --- | --- | --- |
| A | KR 10-2021-0042777 A (SAMSUNG ELECTRONICS CO., LTD. et al.) 20 April 2021 (2021-04-20)<br>See entire document. | 1-12 |

Form PCT/ISA/210 (second sheet) (July 2022)

## INTERNATIONAL SEARCH REPORT
### Information on patent family members

International application No.

**PCT/KR2023/015926**

| Patent document cited in search report | | | Publication date (day/month/year) | Patent family member(s) | | | Publication date (day/month/year) |
|---|---|---|---|---|---|---|---|
| JP | 2020-009203 | A | 16 January 2020 | None | | | |
| KR | 10-2022-0112692 | A | 11 August 2022 | None | | | |
| KR | 10-2021-0047262 | A | 29 April 2021 | EP | 4050612 | A1 | 31 August 2022 |
| | | | | KR | 10-2021-0110539 | A | 08 September 2021 |
| | | | | KR | 10-2296183 | B1 | 01 September 2021 |
| | | | | US | 2022-0383993 | A1 | 01 December 2022 |
| | | | | WO | 2021-080295 | A1 | 29 April 2021 |
| JP | 2021-081769 | A | 27 May 2021 | JP | 7133534 | B2 | 08 September 2022 |
| KR | 10-2021-0042777 | A | 20 April 2021 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20210042777 A

- KR 20210110539 A